# EUROPEAN PATENT APPLICATION

(11) **EP 4 238 989 A1**
(43) Date of publication of application: **06.09.2023**
(21) Application number: 21884838.0
(22) Date of filing: 23.09.2021
(51) Int. Cl.: C07K 16/24, C07K 16/46, C12N 15/13, C12N 15/81, A61K 39/395

(54) **ANTI-IL5 NANOANTIBODY AND USE THEREOF**

(30) Priority: 30.10.2020 CN 202011189604
(71) Applicant: Shanghai Novamab Biopharmaceuticals Co., Ltd., Pudong New Area, Shanghai 201321 (CN)
(72) Inventor: WAN, Yakun, Shanghai 201321 (CN); ZHU, Min, Shanghai 201321 (CN); GAI, Junwei, Shanghai 201321 (CN); LI, Guanghui, Shanghai 201321 (CN); SHEN, Xiaoning, Shanghai 201321 (CN)
(74) Representative: Avidity IP
(86) International application number: PCT/CN2021/119969
(87) International publication number: WO 2022/089108

(57) **Abstract**

Provided are an IL5 nanoantibody and the use thereof. Specifically, provided is an IL5 nanoantibody, and further provided are a coding sequence for coding the above-mentioned nanoantibody, a corresponding expression vector, a host cell capable of expressing the nanoantibody, and a method for producing the nanoantibody. The nanoantibody can specifically recognize IL5 from humans and cynomolgus monkeys, does not recognize IL5 from mice, and has a good binding activity; the nanoantibody has a good IL5/IL5R blocking activity, and the blocking activity is obviously superior to that of a control antibody Nucala; the nanoantibody can effectively inhibit the proliferation of TF-1 cells induced by IL5, and the inhibition activity thereof is superior to that of the control antibody Nucala; and the expression yield of the nanoantibody in Pichia pastoris can reach 13g/L, and the purity of target protein expressed in the supernatant is high.

## Description

### TECHNICAL FIELD

The present invention relates to the technical field of biomedicine or biopharmaceuticals, in particular to an anti-IL5 nanobody and use thereof.

### BACKGROUND

Eosinophils play an important role in protecting the body from infection. But in some individuals, elevated levels of eosinophils may lead to inflammation and play a role in the development of certain inflammatory diseases. IL5 is the known cytokine with the highest selectivity for eosinophils, which can regulate the growth, differentiation, recruitment, activation, and survival of eosinophils. Its receptor, IL5Rα, is highly expressed on eosinophils, which plays a key role in the removal of allergens from blood and tissues by eosinophils. IL5 is currently considered to be one of the key drivers of the Th2 pathway, and the binding of IL5 to its receptor will further activate the downstream JAK-STAT signaling pathway.

In recent years, as an important target in the research and development of immunomodulatory drugs, IL5 has played an important role in the field of immunotherapy. Three monoclonal antibody drugs targeting IL5/IL5Rα have been approved for marketing globally, two of which are IL5 antibodies (Mepolizumab of GSK Company and Reslizumab of Teva Company) and one is IL5Rα antibody (Benralizumab of AstraZeneca Company), providing patients with new treatment options. The IL5 humanized monoclonal antibody injection (610) of Sunshine Guojian, a local Chinese pharmaceutical company, and the IL5 antibody (SHR-1703) of Hengrui Pharmaceuticals have also been approved for clinical trials by the National Medical Products Administration. The world's first approved IL5 monoclonal antibody drug, Mepolizumab of GSK Company, has been approved for a number of clinical studies, targeting moderate asthma, eosinophilic granulomatosis polyangiitis (EGPA), chronic obstructive pulmonary disease (COPD), chronic sinusitis with nasal polyps (CRSwNP), severe hypereosinophilic syndrome, severe specific dermatitis, severe bilateral nasal polyps and other indications.

Up to now, no nanobody drug targeting IL5 has been published in the market. Nanobody (Nb), also known as heavy chain nanobody VHH (variable domain of heavy chain of heavy-chain antibody), is a heavy chain antibody (HCAb) naturally lacking light chain in camels, and the nanobody consisting of only one heavy chain variable region obtained by cloning its variable region is the smallest unit of stable, fully functional, binding antigen currently available. Nanobodies have the characteristics of high stability, good water solubility, simple humanization, high targeting, and strong penetration, and play a huge function beyond imagination in immune experiments, diagnosis and treatment. Nanobodies are gradually becoming an emerging force in the diagnosis and treatment by the new generation antibodies.

Therefore, the development of a new type of anti-IL5 nanobody has a good clinical application prospect, and is expected to fill the gap in the market of IL5 nanobody drugs and benefit patients.

### SUMMARY OF THE INVENTION

The object of the present invention is to provide an anti-IL5 nanobody and use thereof.

In the first aspect of the present invention, it provides an anti-IL5 nanobody, which can specifically bind to IL5, and the complementary determination region (CDR) of the VHH chain in the nanobody is one or more selected from the group consisting of:
(1) CDR1 shown in SEQ ID NO:1, CDR2 shown in SEQ ID NO:2, and CDR3 shown in SEQ ID NO:3;
(2) CDR1 shown in SEQ ID NO:10, CDR2 shown in SEQ ID NO:11, and CDR3 shown in SEQ ID NO:12;
(3) CDR1 shown in SEQ ID NO:19, CDR2 shown in SEQ ID NO:20, and CDR3 shown in SEQ ID NO:21;
(4) CDR1 shown in SEQ ID NO:28, CDR2 shown in SEQ ID NO:29, and CDR3 shown in SEQ ID NO:30.

In another preferred embodiment, any one of the above amino acid sequences further includes a derivative sequence that is optionally added, deleted, modified and/or substituted with at least one (such as 1-3, preferably 1-2, more preferably 1) amino acid and can retain the ability to bind to IL5.

In another preferred embodiment, the VHH chain of the anti-IL5 nanobody further comprises a framework region (FR), and the framework region (FR) is one or more selected from the group consisting of:
(1) FR1 shown in SEQ ID NO:4, FR2 shown in SEQ ID NO:5, FR3 shown in SEQ ID NO:6, and FR4 shown in SEQ ID NO:7;
(2) FR1 shown in SEQ ID NO:13, FR2 shown in SEQ ID NO:14, FR3 shown in SEQ ID NO: 15, and FR4 shown in SEQ ID NO: 16;
(3) FR1 shown in SEQ ID NO:22, FR2 shown in SEQ ID NO:23, FR3 shown in SEQ ID NO:24, and FR4 shown in SEQ ID NO:25;
(4) FR1 shown in SEQ ID NO:31, FR2 shown in SEQ ID NO:32, FR3 shown in SEQ ID NO:33, and FR4 shown in SEQ ID NO:34;
(5) FR1 shown in SEQ ID NO:37, FR2 shown in SEQ ID NO:38, FR3 shown in SEQ ID NO:39, and FR4 shown in SEQ ID NO:40; and
(6) FR1 shown in SEQ ID NO:43, FR2 shown in SEQ ID NO:44, FR3 shown in SEQ ID NO:45, and FR4 shown in SEQ ID NO:46.

In another preferred embodiment, the CDR1, CDR2 and CDR3 are separated by framework regions FR1, FR2, FR3 and FR4.

In another preferred embodiment, the amino acid sequence of the VHH chain of the anti-IL5 nanobody is selected from the group consisting of: SEQ ID NO: 8, SEQ ID NO: 17, SEQ ID NO: 26, SEQ ID NO: 35, SEQ ID NO: 41, SEQ ID NO: 47, and a combination thereof.

In another preferred embodiment, the anti-IL5 nanobody includes monomer, bivalent (bivalent antibody), tetravalent (tetravalent antibody), and/or multivalent (multivalent antibody).

In another preferred embodiment, the anti-IL5 nanobody comprises two VHH chains with amino acid sequences as shown in SEQ ID NO:41 and/or SEQ ID NO:47, preferably, the VHH chains are linked by a linker peptide.

In another preferred embodiment, the linker peptide is selected from the following sequences: (GₐS_{b})ₓ-(GₘSₙ)_{y}, wherein a, b, m, n, x, y = 0 or 1 or 2 or 3 or 4 or 5 or 6 or 7 or 8 or 9 or 10 (preferably, a = 4 and b = 1, m = 3 and n = 1).

In another preferred embodiment, the sequence of the linker peptide is (G₄S)₄.

In another preferred embodiment, the anti-IL5 nanobody can recognize two different IL5 epitopes.

In another preferred embodiment, the nanobody includes a humanized antibody, a camel antibody, a chimeric antibody.

In the second aspect of the present invention, it provides an anti-IL5 antibody, which is an antibody against an interleukin 5 (IL5) epitope and has the anti-IL5 nanobody of the first aspect of the present invention.

In another preferred embodiment, the anti-IL5 antibody includes monomer, bivalent (bivalent antibody), tetravalent (tetravalent antibody), and/or multivalent (multivalent antibody).

In another preferred embodiment, the anti-IL5 antibody comprises one or more VHH chains with amino acid sequences as shown in SEQ ID NO: 8, SEQ ID NO: 17, SEQ ID NO: 26, SEQ ID NO: 35, SEQ ID NO:41 or SEQ ID NO:47.

In another preferred embodiment, the anti-IL5 antibody comprises two VHH chains with amino acid sequences as shown in SEQ ID NO:41 and/or SEQ ID NO:47.

In another preferred embodiment, the structure of the anti-IL5 antibody from the N-terminus to the C-terminus is shown in Formula I:

A1-A2-L-B (I)

; wherein,
"-" is a peptide bond or linker peptide;
A1 and A2 are each independently any one of the anti-IL5 nanobodies of the first aspect of the present invention;
B is the Fc fragment of IgG; and
L is none or a flexible linker.

In another preferred embodiment, the A1 and A2 are each independently a VHH chain with an amino acid sequence as shown in SEQ ID NO:41 or SEQ ID NO:47.

In another preferred embodiment, the A1 is a VHH chain with an amino acid sequence as shown in SEQ ID NO:41, and the A2 is a VHH chain with an amino acid sequence as shown in SEQ ID NO:47.

In another preferred embodiment, the A1 is a VHH chain with an amino acid sequence as shown in SEQ ID NO:47, and the A2 is a VHH chain with an amino acid sequence as shown in SEQ ID NO:41.

In another preferred embodiment, the flexible linker is a linker peptide.

In another preferred embodiment, the VHH chains are linked by a linker peptide.

In another preferred embodiment, the linker peptide is selected from the following sequences: (GₐS_{b})ₓ-(GₘSₙ)_{y}, wherein a, b, m, n, x, y = 0 or 1 or 2 or 3 or 4 or 5 or 6 or 7 or 8 or 9 or 10 (preferably, a = 4 and b = 1, m = 3 and n = 1).

In another preferred embodiment, the sequence of the linker peptide is (G₄S)₄.

In another preferred embodiment, the amino acid sequence of the antibody is shown in SEQ ID NO:53, SEQ ID NO:55, SEQ ID NO:57, or SEQ ID NO:59.

In another preferred embodiment, the antibody can specifically bind to IL5 protein with correct spatial structure.

In another preferred embodiment, the antibody can effectively block the interaction between IL5 and IL5R.

In another preferred embodiment, the antibody can recognize human or cynomolgus macaques IL5, and does not recognize mouse IL5.

In another preferred embodiment, the affinity of the antibody to IL5 is less than 1nM.

In another preferred embodiment, the antibody has good IL5/IL5R blocking activity, and the blocking activity is significantly better than that of the control antibody Nucala. Wherein, the control antibody Nucala is a marketed drug of GlaxoSmithKline, known as Mepolizumab.

In another preferred embodiment, the antibody can effectively inhibit the proliferation of TF-1 cells induced by IL5, and its inhibitory activity is superior to that of the control antibody Nucala.

In another preferred embodiment, the antibody is a nanobody.

In the third aspect of the present invention, it provides an anti-IL5 nanobody Fc fusion protein, and the structure of the fusion protein from N-terminus to C-terminus is as shown in the Formula Ia or Ib:

A- L-B (Ia)

;

B- L-A (Ib)

; wherein,
"-" is a peptide bond;
A is one or more anti-IL5 nanobodies of the first aspect of the present invention;
B is the Fc fragment of IgG; and
L is none or a flexible linker.

In another preferred embodiment, the flexible linker is a linker peptide.

In another preferred embodiment, the Fc fragment of IgG includes the Fc fragment of human IgG.

In another preferred embodiment, the Fc fragment of IgG is selected from the group consisting of Fc fragments of IgG1, IgG2, IgG3, IgG4, and a combination thereof.

In another preferred embodiment, the Fc fragment of IgG is IgG4.

In another preferred embodiment, the amino acid sequence of the Fc fragment is as shown in SEQ ID NO:63.

In another preferred embodiment, the amino acid sequence of the fusion protein is as shown in SEQ ID NO:49, SEQ ID NO:51, SEQ ID NO:53, SEQ ID NO:55, SEQ ID NO:57, or SEQ ID NO:59.

In another preferred embodiment, the fusion protein is a nanobody Fc fusion protein against IL5 epitope.

In the fourth aspect of the present invention, it provides a polynucleotide encoding a protein selected from the group consisting of the anti-IL5 nanobody of the first aspect of the present invention, the anti-IL5 antibody of the second aspect of the present invention, or the anti-IL5 nanobody Fc fusion protein of the third aspect of the present invention.

In another preferred embodiment, the polynucleotide sequence is a combination, preferably, the polynucleotide sequence contains one or more of SEQ ID NO: 9, SEQ ID NO: 18, SEQ ID NO: 27, SEQ ID NO: 36, SEQ ID NO: 42, SEQ ID NO: 48, SEQ ID NO: 50, SEQ ID NO: 52, SEQ ID NO: 54, SEQ ID NO: 56, SEQ ID NO: 58, SEQ ID NO: 60 or SEQ ID NO: 62.

In another preferred embodiment, the polynucleotide includes DNA or RNA.

In the fifth aspect of the present invention, it provides an expression vector comprising the polynucleotide of the fourth aspect of the present invention.

In another preferred embodiment, the expression vector is selected from the group consisting of DNA, RNA, a viral vector, a plasmid, a transposon, other gene transfer system, and a combination thereof.

Preferably, the expression vector comprises a viral vector, such as a lentivirus, an adenovirus, an AAV virus, a retrovirus, and a combination thereof.

In the sixth aspect of the present invention, it provides a host cell comprising the expression vector of the fifth aspect of the present invention, or having the polynucleotide of the fourth aspect of the present invention integrated in the genome.

In another preferred embodiment, the host cell comprises a prokaryotic cell or a eukaryotic cell.

In another preferred embodiment, the host cell is selected from the group consisting of *Escherichia coli,* a yeast cell, a mammalian cell, a bacteriophage, and a combination thereof.

In another preferred embodiment, the prokaryotic cell is selected from the group consisting of *Escherichia coli, Bacillus subtilis,* lactic acid bacteria, *Streptomyces, Proteus mirabilis,* and a combination thereof.

In another preferred embodiment, the eukaryotic cell is selected from the group consisting of *Pichia pastoris, Saccharomyces cerevisiae, Schizosaccharomyces pombe,* Trichoderma, and a combination thereof.

In another preferred embodiment, the host cell is *Pichia pastoris.*

In the seventh aspect of the present invention, it provides a method for producing an anti-IL5 nanobody or Fc fusion protein thereof, which comprises the steps:
(a) culturing the host cell of the sixth aspect of the present invention under conditions suitable for producing the nanobody or Fc fusion protein thereof, thereby obtaining a culture containing the anti-IL5 nanobody or Fc fusion protein thereof;
(b) isolating or recovering the anti-IL5 nanobody or Fc fusion protein thereof from the culture; and
(c) optionally, purifying and/or modifying the anti-IL5 nanobody or Fc fusion protein thereof obtained in step (b).

In the eighth aspect of the present invention, it provides an immunoconjugate comprising:
(a) the anti-IL5 nanobody of the first aspect of the present invention, or the anti-IL5 antibody of the second aspect of the present invention, or the anti-IL5 nanobody Fc fusion protein of the third aspect of the present invention; and
(b) a coupling moiety selected from the group consisting of a detectable label, a drug, a toxin, a cytokine, a radionuclide, an enzyme, a gold nanoparticle/nanorod, a nanomagnetic particle, a viral coat protein or VLP, and a combination thereof.

In another preferred embodiment, the radionuclide comprises:
(i) a diagnostic isotope, which is selected from the group consisting of Tc-99m, Ga-68, F-18, I-123, I-125, I-131, In-111, Ga-67, Cu-64, Zr-89, C-11, Lu-177, Re-188, and a combination thereof; and/or
(ii) a therapeutic isotope, which is selected from the group consisting of Lu-177, Y-90, Ac-225, As-211, Bi-212, Bi-213, Cs-137, Cr-51, Co-60, Dy-165, Er-169, Fm-255, Au-198, Ho-166, I-125, I-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133 Yb-169, Yb-177, and a combination thereof.

In another preferred embodiment, the coupling moiety is a drug or a toxin.

In another preferred embodiment, the drug is a cytotoxic drug.

In another preferred embodiment, the cytotoxic drug is selected from the group consisting of an anti-tubulin drug, a DNA minor groove binding agent, a DNA replication inhibitor, an alkylating agent, an antibiotic, a folate antagonist, an anti-metabolite, a chemotherapeutics sensitizer, a topoisomerase inhibitor, vinca alkaloid, and a combination thereof.

In another preferred embodiment, examples of particularly useful cytotoxic drug classes comprises, for example, a DNA minor groove binding reagent, a DNA alkylation reagent, and a tubulin inhibitor, and a typical cytotoxic drug includes, for example, auristatins, camptothecins, duocarmycins, etoposides, maytansines and maytansinoids (such as DM1 and DM4), taxanes, benzodiazepines or benzodiazepine containing drugs (e.g., pyrrolo [1,4] benzodiazepines (PBDs), indolinobenzodiazepines and oxazolidinobenzodiazepines), vinca alkaloids, and a combination thereof.

In another preferred embodiment, the toxin is selected from the group consisting of: auristatins (e.g, auristatin E, auristatin F, MMAE and MMAF), chlortetracycline, maytansoid, ricin, ricin A-chain, combretastatin, docamicin, dolastatin, doxorubicin, daunorubicin, paclitaxel, cisplatin, cc1065, ethidium bromide, mitomycin, etoposide, tenoposide, vincristine, vinblastine, colchicine, dihydroxyanthraxdione, actinomycin, diphtheria toxin, pseudomonas exotoxin (PE) A, PE40, acacia toxin, acacia A chain, capsule root toxin A chain, α-octococcus, white tree toxin, mitogellin, retstrictocin, phenomycin, enomycin, curicin, curicin, calicheamicin, Sapaonaria officinalis inhibitor, glucocorticoids, and a combination thereof.

In another preferred embodiment, the coupling moiety is a detectable label.

In another preferred embodiment, the coupling moiety is selected from the group consisting of: a fluorescent or luminescent label, a radioactive label, MRI (magnetic resonance imaging) or CT (electronic computer tomography) contrast agent, or an enzyme capable of producing detectable products, a radionuclide, a biological toxin, a cytokine (such as IL-2), an antibody, an antibody Fc fragment, an antibody scFv fragment, a gold nanoparticle/nanorod, a viral particle, a liposome, a nanomagnetic particle, a prodrug activating enzyme (such as DT-cardiomyolase (DTD) or biphenyl hydrolase-like protein (BPHL)), or a nanoparticle in any form.

In the ninth aspect of the present invention, it provides a pharmaceutical composition containing:
(i) the anti-IL5 nanobody of the first aspect of the present invention, or the anti-IL5 antibody of the second aspect of the present invention, or the anti-IL5 nanobody Fc fusion protein of the third aspect of the present invention, or the immunoconjugate of the eighth aspect of the present invention; and
(ii) a pharmaceutically acceptable carrier.

In another preferred embodiment, the conjugate moiety of the immunoconjugate is a drug, a toxin, and/or a therapeutic isotope.

In another preferred embodiment, the pharmaceutical composition further contains other drugs for treating asthma, atopic dermatitis, arthritis, allergic rhinitis and/or eczema, such as corticosteroids (TCS), sodium nedolomide, sodium cromolyn, theophylline, leukotriene receptor antagonist, and a combination thereof.

In another preferred embodiment, the pharmaceutical composition is used to prepare a drug for preventing and treating a disease or condition associated with IL5/IL5R signaling.

In the tenth aspect of the present invention, it provides a use of the anti-IL5 nanobody of the first aspect of the present invention, the anti-IL5 antibody of the second invention of the present invention, the anti-IL5 nanobody Fc fusion protein of the third aspect of the present invention, or the immunoconjugate of the eighth aspect of the present invention; (a) for the preparation of drugs for preventing and/or treating diseases or conditions related to IL5/IL5R signaling; (b) for the preparation of a reagent, a test plate or a kit for detecting IL5.

In another preferred embodiment, the diseases or conditions include but are not limited to: asthma, atopic dermatitis, arthritis, allergic rhinitis, eczema, sinusitis, nasal polyps, chronic obstructive pulmonary disease, eosinophilic granulomatosis polyangiitis, hypereosinophilic syndrome, and a combination thereof.

In another preferred embodiment, the IL5 is human IL5.

In another preferred embodiment, the reagent is a diagnostic reagent.

In another preferred embodiment, the diagnostic reagent is a contrast agent.

In another preferred embodiment, the reagent is used to detect IL5 protein or its fragments in a sample.

In the eleventh aspect of the present invention, it provides a multispecific antibody, which comprises: the anti-IL5 nanobody of the first aspect of the present invention, or the anti-IL5 antibody of the second aspect of the present invention.

In another preferred embodiment, the multispecific antibody further comprises a second antigen-binding region targeting a target selected from the group consisting of IL-4R, IL-4Rα, IL-13, IL-13R, IL-11, IL-11R, and a combination thereof.

In another preferred embodiment, the second antigen-binding region is a nanobody.

In another preferred embodiment, the multispecific antibody comprises one or more second antigen-binding regions.

In another preferred embodiment, the multispecific antibody further comprises an Fc segment of the antibody.

In the twelfth aspect of the present invention, it provides a recombinant protein having:
(i) the anti-IL5 nanobody of the first aspect of the present invention, or the anti-IL5 antibody of the second aspect of the present invention, or the anti-IL5 nanobody Fc fusion protein of the third aspect of the present invention; and
(ii) optionally a tag sequence assisting expression and/or purification.

In another preferred embodiment, the tag sequence includes an Fc tag, an HA tag and a 6His tag.

In another preferred embodiment, the recombinant protein specifically binds to IL5 protein.

In the thirteenth aspect of the present invention, it provides a use of the anti-IL5 nanobody of the first aspect of the present invention, or the anti-IL5 antibody of the second invention of the present invention, or the anti-IL5 nanobody Fc fusion protein of the third aspect of the present invention, or the immunoconjugate of the eighth aspect of the present invention, for detecting IL5 protein in a sample, or for treating and/or preventing diseases or conditions related to IL5/IL5R signaling.

In another preferred embodiment, the diseases or conditions include but are not limited to: asthma, atopic dermatitis, arthritis, allergic rhinitis, eczema, sinusitis, nasal polyps, chronic obstructive pulmonary disease, eosinophilic granulomatosis polyangiitis, hypereosinophilic syndrome, and a combination thereof.

In another preferred embodiment, the detection comprises a flow cytometry detection and a cellular immunofluorescence detection.

In another preferred embodiment, the use is diagnostic and/or non-diagnostic, and/or therapeutic and/or non-therapeutic.

In the fourteenth aspect of the present invention, it provides a method for detecting IL5 protein in a sample, which comprises the steps:
(1) contacting the anti-IL5 nanobody of the first aspect of the present invention, or the anti-IL5 antibody of the second aspect of the present invention, or the anti-IL5 nanobody Fc fusion protein of the third aspect of the present invention, or the immunoconjugate of the eighth aspect of the present invention with a sample; and
(2) detecting the formation of an antigen-antibody complex, wherein the formation of a complex indicates the presence of IL5 protein in the sample.

In another preferred embodiment, the method is a non-diagnostic and non-therapeutic method.

In the fifteenth aspect of the present invention, it provides an IL5 protein detection reagent comprising:
(i) the anti-IL5 nanobody of the first aspect of the present invention, or the anti-IL5 antibody of the second aspect of the present invention, or the anti-IL5 nanobody Fc fusion protein of the third aspect of the present invention, or the immunoconjugate of the eighth aspect of the present invention; and
(ii) an acceptable carrier for detection.

In another preferred embodiment, the coupling moiety of the immunoconjugate is a diagnostic isotope.

In another preferred embodiment, the detectably acceptable carrier is a non-toxic, inert, aqueous carrier medium.

In another preferred embodiment, the detection reagent is one or more reagents selected from the group consisting of an isotope tracer, a contrast agent, a flow detection reagent, a cellular immunofluorescence detection reagent, a magnetic nanoparticle and an imaging agent.

In another preferred embodiment, the detection reagent is used for *in vivo* detection.

In another preferred embodiment, the dosage form of the detection reagent is liquid or powder (e.g., aqua, injection, lyophilized powder, tablet, buccal, inhaler).

In the sixteenth aspect of the present invention, it provides a kit for detecting IL5 protein, which comprises the immunoconjugate of the eighth aspect of the present invention or the detection reagent of the fifteenth aspect of the present invention, and instructions.

In another preferred embodiment, the instructions describe that the kit is used for non-invasively detecting the IL5 expression of the subject to be tested.

In the seventeenth aspect of the present invention, it provides a use of the immunoconjugate of the eighth aspect of the present invention for preparing a contrast agent for detecting IL5 protein in vivo.

In another preferred embodiment, the detection is used for the diagnosis or prognosis of asthma, atopic dermatitis, arthritis, allergic rhinitis, eczema, sinusitis, nasal polyps, chronic obstructive pulmonary disease, eosinophilic granulomatosis polyangiitis, hypereosinophilic syndrome, etc.

It should be understood that within the scope of the present invention, each technical features of the present invention described above and in the following (such as examples) may be combined with each other to form a new or preferred technical solution, which is not listed here due to space limitations.

### DESCRIPTION OF THE DRAWINGS

Figure 1 shows the process of IL5 nanobody screening and enrichment. After five rounds of panning, 120-fold and 14.6-fold enrichment of IL5-specific nanobody phage appeared in the two libraries, respectively.
Figure 2 shows the results of a preliminary flow cytometry screening for nanobodies that block the interaction of IL5 with IL5R. The results show that 11 of the 96 nanobodies have blocking activity.
Figure 3 shows the species cross-activity results of 11 blocking IL5 nanobodies detected by ELISA. The results show that 11 nanobodies can recognize human and cynomolgus macaques IL5, but can not recognize mouse IL5.
Figure 4 shows the results of the affinity of nanobodies detected by using the Bio-Layer Interferometry Fortebio. The results show that the affinity of 11 nanobodies to IL5 is less than 1nM.
Figure 5 shows the results of the binding activity of nanobodies detected by ELISA. The results show that the binding activity of the four nanobodies is better than that of the control antibody Nucala.
Figure 6 shows the blocking activity of candidate IL5 nanobodies identified by flow cytometry. The results show that all the four nanobodies have good IL5/IL5R blocking activity, and the blocking activity of Nb21 and Nb66 is significantly better than that of the control antibody Nucala.
Figure 7 shows the results of antigen recognition epitope consistency of blocking nanobodies detected by ELISA. The results show that Nb21 and Nb66 are different in antigen recognition epitopes.
Figure 8 shows the blocking activity of humanized bivalent nanobodies detected by flow cytometry. The results show that the activity of the humanized bivalent antibody is significantly higher than that of the monovalent antibody, and the blocking activity of the HuNb21-HuNb66-Fc and HuNb66-HuNb21-Fc bivalent biepitope antibodies is the best, and is significantly better than that of the control antibody Nucala.
Figure 9 shows the results of the content of bivalent biepitopic IL5 nanobodies in Pichia pastoris fermentation supernatant detected by Fortebio. The results show that under the fermentation conditions, the expression of bivalent biepitopic antibody increased with the extension of culture time, and the antibody yield could reach 13g/L after 202 hours of fermentation culture.
Figure 10 shows the results of SDS-PAGE detection of the biepitopic bivalent IL5 nanobody in Pichia fermentation supernatant. The results show that the expression yield of the biepitopic IL5 nanobody HuNb66-HuNb21 in Pichia pastoris can reach 13g/L, and the purity of the target protein expression supernatant is high.
Figure 11 shows the results of the blocking activity of the bivalent biepitopic IL5 nanobody detected by flow cytometry. The results show that the blocking activity of the bivalent biepitopic antibody HuNb66-HuNb21 expressed by yeast (IC₅₀= 0.841 µg/mL) is better than that of the control antibody Nucala (IC₅₀=2.035 µg/mL).
Figure 12 shows the detection results of the proliferation inhibitory effect of the bivalent biepitope IL5 nanobody on TF1 cells. The results show that the bivalent biepitope antibody expressed by yeast can effectively inhibit the proliferation of TF-1 cells induced by IL5, and its inhibitory activity (IC₅₀ HuNb66-HuNb21 = 0.0512 nM) is better than that of the control antibody on the proliferation of TF-1 cells (IC₅₀ Nucala = 0.1782 nM).

### Detailed Description

After extensive and in-depth research, and a large number of screening, the present inventors unexpectedly found a class of IL5 nanobodies for the first time. The experimental results show that the nanobody of the present invention can specifically recognize human and Cynomolgus macaques IL5, do not recognize mouse IL5, with good specificity and binding activity. The nanobody of the present invention has good IL5/IL5R blocking activity, and the blocking activity is significantly superior to the control antibody Nucala. The nanobody of the present invention can effectively inhibit the proliferation of TF-1 cells induced by IL5, and its inhibitory activity is better than that of the control antibody Nucala. The expression yield of the nanobody of the present invention in Pichia pastoris can reach 13g/L, and the purity of the target protein expression supernatant is high.

### Term

As used herein, the terms "nanobody of the present invention", "the nanobody of the present invention", "anti-IL5 nanobody of the present invention", "IL5 nanobody of the present invention", "anti-IL5 nanobody", "IL5 nanobody" have the same meaning and can be used interchangeably to refer to a nanobody that specifically recognizes and binds to IL5, including human IL5.

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a plurality of constant regions. Each light chain has a variable region (VL) at one end and a constant region at the other end; the constant region of light chain pairs with the first constant region of heavy chain, and the variable region of light chain pairs with the variable region of heavy chain. Special amino acid residues form an interface between the variable regions of a light chain and a heavy chain.

As used herein, the terms "single domain antibody", "VHH", "nanobody" and "heavy chain antibody" have the same meaning and can be used interchangeably to refer to cloning the variable region of the heavy chain of the antibody, constructing a nanobody (VHH) composed of only one heavy chain variable region, which is the smallest antigen-binding fragment with complete function. Usually, the antibody with natural deletion of light chain and heavy chain constant region 1(CH1) is obtained first, and then the variable region of the antibody heavy chain is cloned to construct a nanobody (VHH) composed of only one heavy chain variable region.

As used herein, the term "variable" means that certain portion of the variable region in an antibody differ in sequence, which is responsible for the binding and specificity of various specific antibodies to their specific antigen. However, the variability is not distributed evenly throughout the variable regions of an antibody. It is concentrated in three fragments called complementarity determination regions (CDRs) or hypervariable regions in light chain and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partical β-sheet structure. The CDRs in each chain are closely linked together via the FR regions, and together with the CDRs of the other chain, form the antigen binding site of an antibody (see Kabat et al., NIH Publ. No. 91-3242, Volume I, pages 647-669 (1991)). Constant regions are not directly involved in the binding of antibodies to antigen, however, they exhibit different effector functions, such as participating in the antibody-dependent cytotoxicity of antibodies.

As known to those skilled in the art, an immunoconjugates and the fusion expression product includes: a drug, a toxin, a cytokine, a radionuclide, an enzyme and other diagnostic or therapeutic molecules that bind to the antibody or fragment thereof of the present invention to form a conjugate. The invention also includes a cell surface marker or antigen that binds to the anti-IL5 antibody or fragment thereof.

As used herein, the terms "heavy chain variable region" and "VH" can be used interchangeably.

As used herein, the terms "variable region" and "complementarity determine region (CDR)" can be used interchangeably.

In a preferred embodiment of the present invention, the heavy chain variable region of the antibody comprises three complementarity determining regions, CDR1, CDR2, and CDR3.

In a preferred embodiment of the present invention, the heavy chain of the antibody comprises the above-mentioned heavy chain variable region and the heavy chain constant region.

In the present invention, the terms "antibody of the present invention", "protein of the present invention", or "polypeptide of the present invention" may be used interchangeably and refer to a polypeptide that specifically binds to IL5 protein, such as a protein or polypeptide having a heavy chain variable region. They can contain or do not contain starting methionine.

The invention also provides other proteins or fusion expression products having the antibody of the present invention. Specifically, the present invention includes any protein or protein conjugate and fusion expression product (i.e., immunoconjugate and fusion expression product) having a heavy chain containing variable regions, as long as the variable region is the same as or has at least 90% homology with the variable regions of the heavy chain of the antibody of the present invention, preferably at least 95% homology.

In general, the antigen binding characteristics of an antibody can be described by three specific regions located in the heavy chain variable region, called the variable region (CDR), which are separated into four frame regions (FR). The amino acid sequence of the four FRs is relatively conservative and does not directly participate in the binding reaction. These CDRs form a loop structure, and the β-sheets formed by the FRs in between are spatially close to each other, and the CDRs on the heavy chain and the CDRs on the corresponding light chain constitute the antigen-binding site of the antibody. It can be determined which amino acids constitute the FR or CDR region by comparing the amino acid sequences of antibodies of the same type.

The variable regions of the heavy chains of the antibody of the present invention are of particular interest because at least part of them involve binding antigens. Therefore, the present invention includes those molecules with a CDR-bearing antibody heavy chain variable region, as long as their CDR has more than 90% (preferably more than 95%, most preferably more than 98%) homology with the CDR identified here.

The present invention includes not only intact antibodies, but also fragments of immunologically active antibodies or fusion proteins formed by antibodies with other sequences. Thus, the present invention also includes fragments, derivatives and analogs of the antibody.

As used herein, the terms "fragment", "derivative" and "analog" refer to a polypeptide that substantially retain the same biological function or activity of the antibody of the present invention. The polypeptide fragment, derivative or analog of the present invention may be (i) a polypeptide with one or more conservative or non-conservative amino acid residues (preferably conservative amino acid residues) substituted, and such substituted amino acid residues may or may not be encoded by the genetic code, or (ii) a polypeptide with a substituent group in one or more amino acid residues, or (iii) a polypeptide formed by fusion of a mature polypeptide with another compound (such as a compound that extends the half-life of the polypeptide, such as polyethylene glycol), or (iv) a polypeptide formed by fusion of an additional amino acid sequence to the polypeptide sequence (such as a leader sequence or secretory sequence or sequence or protein sequence used to purify the polypeptide, or a fusion protein formed with a 6His tag). According to the teachings herein, these fragments, derivatives and analogs are within the scope of well-known to those skilled in the art.

The antibody of the present invention refers to a polypeptide having IL5 binding activity and comprising the above-mentioned CDR regions. The term also includes variant forms of polypeptides comprising the CDR regions described above that have the same function as the antibody of the present invention. These variants include (but are not limited to): deletion, insertion and/or substitution of one or more (usually 1-50, preferably 1-30, more preferably 1-20, most preferably 1-10) amino acids, and addition of one or more (usually within 20, preferably within 10, more preferably within 5) amino acids at the C- terminal and/or N-terminal. For example, in the art, substitutions with amino acids of similar properties generally do not alter the function of the protein. For another example, addition of one or more amino acids to the C-terminal and/or N-terminal usually does not alter the function of the protein. The term also includes active fragments and active derivatives of the antibody of the present invention.

The variant forms of the polypeptide include homologous sequences, conservative variants, alleles, natural mutants, induced mutants, proteins encoded by DNA capable of hybridizing with the coding DNA of the antibody of the present invention under high or low tightness conditions, and polypeptides or proteins obtained by using anti-serum against the antibody of the present invention.

The present invention also provides other polypeptides, such as fusion proteins containing nanobodies or fragments thereof. In addition to the almost full-length polypeptide, the present invention also includes fragments of the nanobody of the present invention. Typically, the fragment has at least about 50 contiguous amino acids, preferably at least about 50 contiguous amino acids, more preferably at least about 80 contiguous amino acids, and most preferably at least about 100 contiguous amino acids of the antibody of the present invention.

In the present invention, "conservative variant of the antibody of the present invention" refers to a polypeptide formed by replacing at most 10, preferably at most 8, more preferably at most 5, and most preferably at most 3 amino acids with amino acids of similar properties as compared with the amino acid sequence of the antibody of the present invention. These conservative variant polypeptides are best produced by amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gin |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides a polynucleotide molecule encoding the above antibody or fragment thereof or fusion protein thereof. The polynucleotide of the present invention may be in the form of DNA or RNA. DNA form includes cDNA, genomic DNA, or synthetic DNA. DNA may be single-stranded or double-stranded. DNA may be a coding strand or a non-coding strand.

The polynucleotide encoding the mature polypeptide of the present invention includes: the coding sequence that encodes only the mature polypeptide; the coding sequence of the mature polypeptide and various additional coding sequences; the coding sequence of the mature polypeptide (and optional additional coding sequence) and the non-coding sequence.

The term "polynucleotide encoding a polypeptide" may be a polynucleotide that includes sequence encoding the polypeptide, or a polynucleotide that also includes additional coding and/or non-coding sequences.

The present invention also relates to a polynucleotide that hybridize to the above-mentioned sequence and have at least 50%, preferably at least 70%, and more preferably at least 80% identity between the two sequences. In particular, the present invention relates to a polynucleotide that is hybridizable to the polynucleotide of the present invention under strict conditions. In the present invention, "strict conditions" refers: (1) hybridization and elution at lower ionic strength and higher temperature, such as 0.2 × SSC,0.1% SDS, 60 °C; or (2) hybridiztion with denaturing agent, such as 50%(v/v) formamide, 0.1% calf serum/0.1% Ficoll, 42 °C, etc.; or (3) hybridization occurs only when the identity between the two sequences is at least 90% or more, more preferably 95% or more. Furthermore, the polypeptide encoded by the hybridizable polynucleotide has the same biological function and activity as the mature polypeptide.

The full-length nucleotide sequence or fragments of the antibody of the present invention may generally be obtained by PCR amplification, recombination or artificial synthesis methods. A feasible method is to synthesize the relevant sequence by artificial synthesis, especially when the fragment length is short. Generally, fragments with a long sequence can be obtained by first synthesizing multiple small fragments followed by ligation. In addition, the coding sequence of the heavy chain and the expression tag (such as 6His) can be fused together to form a fusion protein.

Once the relevant sequence is obtained, the recombination method can be used to obtain the relevant sequence in large quantities. This is usually to clone it into a vector, then transfer it into a cell, and then separate the relevant sequence from the proliferated host cell by conventional methods. The biomolecules (nucleic acids, proteins, etc.) involved in the present invention include biomolecules in isolated form.

At present, the DNA sequence encoding the protein (or its fragment, or its derivative) of the present invention can be obtained completely by chemical synthesis. The DNA sequence can then be introduced into various existing DNA molecules (or, for example, vectors) and cells known in the art. In addition, mutations can be introduced into the protein sequence of the present invention by chemical synthesis.

The present invention also relates to a vector comprising the appropriate DNA sequence as described above and an appropriate promoter or control sequence. These vectors can be used to transform appropriate host cells to enable them to express proteins.

Host cells may be prokaryotic cells, such as bacterial cells; or lower eukaryotic cells, such as yeast cells; or higher eukaryotic cells, such as mammalian cells. Representative examples include: Escherichia coli, Streptomyces; bacterial cells of Salmonella typhimurium; fungal cells such as yeast; insect cells of Drosophila S2 or Sf9; animal cells of CHO, COS7, 293 cells, etc.

Transformation of host cells with recombinant DNA can be carried out using conventional techniques well known to those skilled in the art. When the host is a prokaryotic organism such as Escherichia coli, the competent cells capable of absorbing DNA can be harvested after the exponential growth period and treated with CaCl₂, the steps used are well known in the art. Another method is to use MgCl₂. If necessary, the transformation can also be carried out by electroporation. When the host is eukaryotic, the following DNA transfection methods can be used: calcium phosphate co-precipitation method, conventional mechanical methods such as microinjection, electroporation, liposome packaging, etc.

The obtained transformant can be cultured by conventional methods to express the polypeptide encoded by the gene of the present invention. Depending on the host cell used, the medium used in the culture may be selected from a variety of conventional medium. Culture is carried out under conditions suitable for host cell growth. When the host cells grow to an appropriate cell density, the selected promoter is induced by a suitable method (such as temperature conversion or chemical induction), and the cells are cultured for a period of time.

The recombinant polypeptide in the above method may be expressed in the cell, or on the cell membrane, or secreted outside the cell. If necessary, the recombinant protein can be isolated and purified by various separation methods using its physical, chemical and other properties. These methods are well known to those skilled in the art. Examples of these methods include, but are not limited to, conventional renaturation treatment, treatment with a protein precipitant (salting-out method), centrifugation, osmotic breakage, ultra-treatment, ultra-centrifugation, molecular sieve chromatography (gel filtration), adsorption chromatography, ion exchange chromatography, high performance liquid chromatography (HPLC) and other liquid chromatography techniques and combinations of these methods.

The antibody of the present invention can be used alone, or can be combined or coupled with a detectable label (for diagnostic purposes), a therapeutic agent, a PK (protein kinase) modifying moietiy, or any combination of these substances.

A detectable marker for diagnostic purposes includes, but is not limited to, a fluorescent or luminescent label, a radioactive label, a MRI (magnetic resonance imaging) or CT (electronic computer tomography) contrast agent, or an enzyme capable of producing a detectable product.

A therapeutic agent that can bind or couple with the antibody of the present invention includes, but is not limited: 1. a radionuclide; 2. a biological toxin; 3. A cytokine such as IL-2, etc; 4. a gold nanoparticle/nanorod; 5. a viral particle; 6. a liposome; 7. a nanomagnetic particle; 8. a prodrug-activating enzyme (e. g., DT-myoflavase (DTD) or biphenyl hydrolase-like protein (BPHL)).

### Interleukin-5 (IL5)

Interleukin (IL5) is the known cytokine with the highest selectivity for eosinophils, which can regulate the growth, activation, survival and migration of eosinophils. Interleukin-5 exerts its proliferative and differentiating effects through the receptor containing interleukin-5 specific α and common β-subunits. IL5 plays a critical role in the migration of eosinophils from the bone marrow to the lung and other organs. IL5 signaling maintains B cell and eosinophil survival and function through JAK-STAT, Btk, and Ras/Raf-ERK signaling. Overexpression of IL5 *in vivo* can significantly increase numbers of eosinophils and B cells, while mice lacking IL5 or IL5 receptor functional genes show many developmental and functional impairments in B cell and eosinophil lines. In humans, the biological effects of IL5 are best characterized by eosinophils. IL5 is currently considered to be one of the key drivers of the Th2 pathway.

### Interleukin-5 Receptor Alpha (IL5Rα)

The receptor of IL5, IL5Rα, is highly expressed on eosinophils, which plays a key role in the removal of allergens from blood and tissues by eosinophils. The IL5 receptor consists of α and β c chains, in which the α subunit is specific for the IL5 molecule, while the β c subunit is also recognized byinterleukin-3(IL3) and granulocyte-macrophage colony-stimulating factor (GM-CSF). The expression of IL5Rα in activated B cells is regulated by a variety of transcription factors, including E12, E47, Sp1, c/EBP β, and Oct2.

### Pharmaceutical composition

The present invention also provides a composition. Preferably, the composition is a pharmaceutical composition comprising the above-mentioned antibody or active fragment thereof or fusion protein thereof, and a pharmaceutically acceptable carrier. Typically, these substances may be formulated in a non-toxic, inert and pharmaceutically acceptable aqueous carrier medium, wherein the pH is typically about 5-8 and preferably about 6-8, although the pH may vary depending on the nature of the substance being formulated and the condition to be treated. The formulated pharmaceutical composition may be administered by conventional routes, including (but not limited to) intraperitoneal, intravenous, or topical administration.

The pharmaceutical composition of the present invention can be directly used to bind IL5 protein molecules, and thus can be used to treat asthma, atopic dermatitis, arthritis, allergic rhinitis, eczema, etc. In addition, other therapeutic agents may be used at the same time.

The pharmaceutical composition of the present invention contains a safe and effective amount (e.g., 0.001-99wt%, preferably 0.01-90wt%, more preferably 0.1-80wt%) of the above-mentioned nanobody of the present invention (or the conjugate thereof) and a pharmaceutically acceptable carrier or excipient. Such carriers include, but are not limited to, saline, buffer, glucose, water, glycerol, ethanol, and a combination thereof. The pharmaceutical formulation should match the mode of administration. The pharmaceutical composition of the present invention may be prepared in the form of an injection, for example, by conventional methods using normal saline or aqueous solutions containing glucose and other adjuvants. The pharmaceutical composition such as an injection and solution should be manufactured under sterile conditions. The dosage of the active ingredient is a therapeutically effective amount, for example, about 10 µg/kg body weight per day to about 50 mg/kg body weight. In addition, the polypeptide of the present invention may also be used with other therapeutic agents.

When a pharmaceutical composition is used, a safe and effective amount of the immune conjugate is administered to a mammal, wherein the safe and effective amount is typically at least about 10 µg/kg body weight, and in most cases no more than about 50 mg/kg body weight, preferably about 10 µg/kg body weight to about 10 mg/kg body weight. Of course, the specific dosage should also consider factors such as the administration route and the patient's health status, which are all within the skill range of a skilled physician.

### Anti-IL5 nanobody

In the present invention, the amino acid sequence of the VHH chain of the anti-IL5 nanobody is selected from one or more of SEQ ID NO: 8, SEQ ID NO: 17, SEQ ID NO: 26, SEQ ID NO: 35, SEQ ID NO: 41, SEQ ID NO: 47.

In a preferred embodiment of the present invention, the anti-IL5 nanobody includes monomer, bivalent (bivalent antibody), tetravalent (tetravalent antibody), and/or multivalent (multivalent antibody).

Typically, the anti-IL5 nanobody comprises two VHH chains with amino acid sequences as shown in SEQ ID NO:41 and/or SEQ ID NO:47.

In another preferred embodiment, the VHH chains are linked by a linker peptide.

In another preferred embodiment, the linker peptide is selected from the following sequences: (GₐS_{b})ₓ-(GₘSₙ)_{y}, wherein a, b, m, n, x, y = 0 or 1 or 2 or 3 or 4 or 5 or 6 or 7 or 8 or 9 or 10 (preferably, a = 4 and b = 1, m = 3 and n = 1).

In another preferred embodiment, the sequence of the linker peptide is (G₄S)₄.

### Labeled Nanobody

In a preferred embodiment of the present invention, the nanobody carries a detectable label. More preferably, the label is selected from the group consisting of an isotope, a colloidal gold label, a colored label or a fluorescent label.

Colloidal gold labeling may be carried out using methods known to those skilled in the art. In a preferred embodiment of the present invention, the IL5 nanobody is labeled with colloidal gold to obtain a colloidal gold labeled nanobody.

### Detection method

The present invention also relates to a method for detecting IL5 protein. The steps of the method are roughly as follows: obtaining a cell and/or tissue sample; dissolving the sample in a medium; and detecting the level of IL5 protein in the dissolved sample.

In the detection method of the present invention, the sample used is not particularly limited, and a representative example is a cell-containing sample present in a cell preservation solution.

### Kit

The present invention also provides a kit containing the antibody (or fragment thereof) or the detection plate of the present invention. In a preferred embodiment of the present invention, the kit further comprises a container, instructions for use, and a buffer, etc.

The present invention also provides a detection kit for detecting the IL5 level, which comprises an antibody that recognizes IL5 proteins, a lysis medium for dissolving a sample, a common reagent and buffer required for detection, such as various buffers, detection labels, detection substrates, etc. The detection kit may be an *in vitro* diagnostic device.

### Application

As described above, the nanobody of the present invention has a wide range of biological application value and clinical application value, and its application relates to the diagnosis and treatment of the IL5 related diseases, basic medical research, biological research and other fields. One preferred application is for clinical diagnosis and targeted therapy for IL5.

**The main advantages of the present invention include:**
(a) The nanobody of the present invention is able to effectively block the interaction of IL5 with IL5R.
(b) The nanobody of the present invention can recognize human, Cynomolgus macaques IL5 and do not recognize mouse IL5.
(c) The nanobody of the present invention has stronger binding activity and blocking activity than the control antibody Nucala.
(d) The nanobody of the present invention can be expressed in Pichia pastoris, with an expression yield of up to 13g/L, and the purity of the target protein expression supernatant is high.
(e) The nanobody of the present invention can effectively inhibit the proliferation of TF-1 cells induced by IL5, and the inhibitory effect is better than that of the control antibody Nucala.

The present invention is further illustrated by the following specific examples. It should be understood that these examples are only for illustrating the present invention and not intend to limit the scope of the present invention. The conditions of the experimental methods not specifically indicated in the following examples are usually in accordance with conventional conditions as described in Sambrook and Russell et al., Molecular Cloning: A Laboratory Manual (third edition)(2001) (CSHL press), or according to the conditions recommended by the manufacturer. Unless otherwise stated, percentages and parts are calculated by weight.

### Example 1: Screening IL5 specific nanobodies by phage display technology

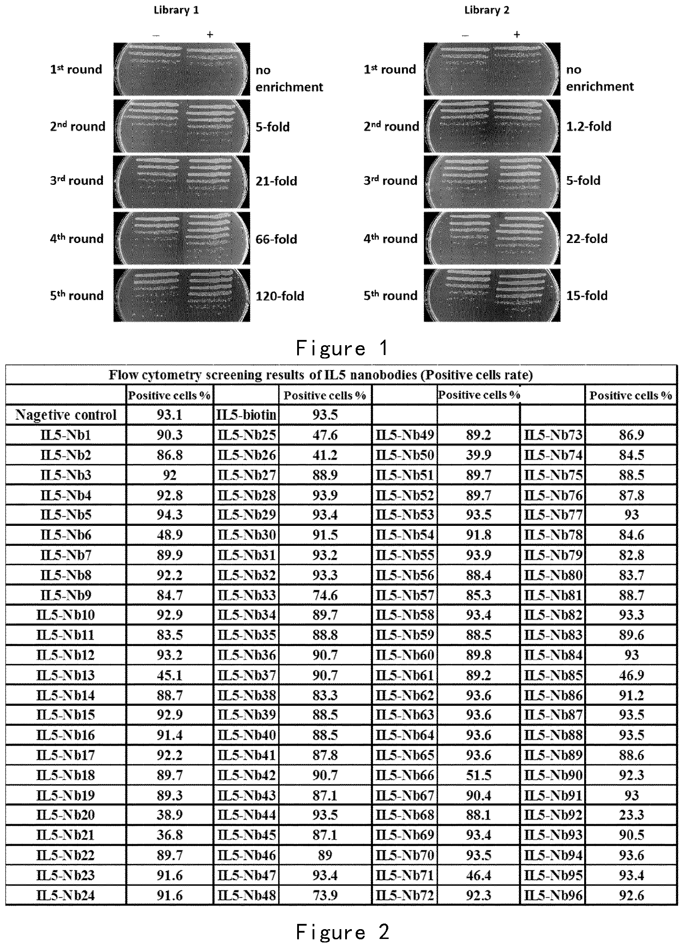

In the early stage, high purity human IL5 protein was mixed with immune adjuvant, and 2 Xinjiang Bactrian camels were immunized. After 7 times immunizations, peripheral blood was collected and lymphocytes were subsequently extracted to isolate RNA. After reverse transcription of RNA, the VHH gene was isolated by nested PCR amplification, and the VHH fragment was cloned into pMECS vector, and then electrotransformed into TG1 competent cells to establish an anti-IL5 nanobody phage display library. The storage capacity of the two libraries was determined to be 6.4 × 10⁸ CFU and 1.3 × 10⁸ CFU, respectively, and the insertion rate of the target fragment VHH in the library was 91.7% and 100%, respectively. Subsequently, the phage display technology was used to screen IL5 specific nanobodies, and the specific screening method is shown in the scheme in Example 3 of patent CN110144011B. After 5 rounds of "bind-wash-elute" enrichment process, 120-fold and 14.6-fold specific phage enrichment were finally obtained, respectively (Figure 1). After PE-ELISA identification and sequencing analysis, 96 strains of IL5 specific nanobodies with different sequences were finally obtained.

### Example 2: Screening of Blocking IL5 Nanobody

IL5 nanobody clone strains with different sequences were inoculated into 1 mL TB medium containing ampicillin with appropriate concentration, cultured in a constant temperature shaker at 37°C to logarithmic growth phase, at that time, IPTG inducer was added to induce at 28°C for 16 hours. After 16h, the thallus was broken by osmotic shock method to obtain crude nanobody extract. 1E6 HEK293F/IL5Ra cells were resuspended in 0.5% BSA-PBS buffer for each sample, 200µL of the above IL5 nanobody crude extract was added respectively, and a negative control (lysate) was set. All samples were added with ILS-Biotin of appropriate concentration and incubated at 4°C for 20 minutes. The cells were washed twice with 1 × PBS, and added with SA-PE, incubated in the dark at 4°C for 20 minutes. The cells were washed twice with PBS, and detected with flow cytometry (BD Caliber). The results are shown in Figure 2: 11 strains of IL5 nanobodies with good blocking effect were initially screened, in which the lower the positive cell percentage value, the better the antibody blocking effect. Among them, the 11 strains of nanobodies were numbered Nb6, Nb13, Nb20, Nb21, Nb25, Nb26, Nb50, Nb66, Nb71, Nb85, Nb92. According to sequence analysis, 11 blocking IL5 nanobody sequences can be divided into 4 families, and the subsequent key research objects are shown in Table 1.

**Table 1 Amino acid and base sequences of 4 blocking nanobodies**

| Anti body No. | Amino acid sequence of variable region | | | Amino acid sequence of framework region | | | | Full-length amino acid sequence | Full-lengt h base sequence |
|---|---|---|---|---|---|---|---|---|---|
| | CDR1 | CDR2 | CDR3 | FR1 | FR2 | FR3 | FR4 | | |
| Nb21 | SEQ ID NO:1 | SEQ ID NO:2 | SEQ ID NO:3 | SEQ ID NO:4 | SEQ ID NO:5 | SEQ ID NO:6 | SEQ ID NO:7 | SEQ ID NO:8 | SEQ ID NO:9 |
| Nb25 | SEQ ID NO:10 | SEQ ID NO:11 | SEQ ID NO:12 | SEQ ID NO:13 | SEQ ID NO:14 | SEQ ID NO:15 | SEQ ID NO:16 | SEQ ID NO:17 | SEQ ID NO:18 |
| Nb66 | SEQ ID NO:19 | SEQ ID NO:20 | SEQ ID NO:21 | SEQ ID NO:22 | SEQ ID NO:23 | SEQ ID NO:24 | SEQ ID NO:25 | SEQ ID NO:26 | SEQ ID NO:27 |
| Nb92 | SEQ ID NO:28 | SEQ ID NO:29 | SEQ ID NO:30 | SEQ ID NO:31 | SEQ ID NO:32 | SEQ ID NO:33 | SEQ ID NO:34 | SEQ ID NO:35 | SEQ ID NO:36 |

### Example 3: Species-specific detection of IL5 nanobodies

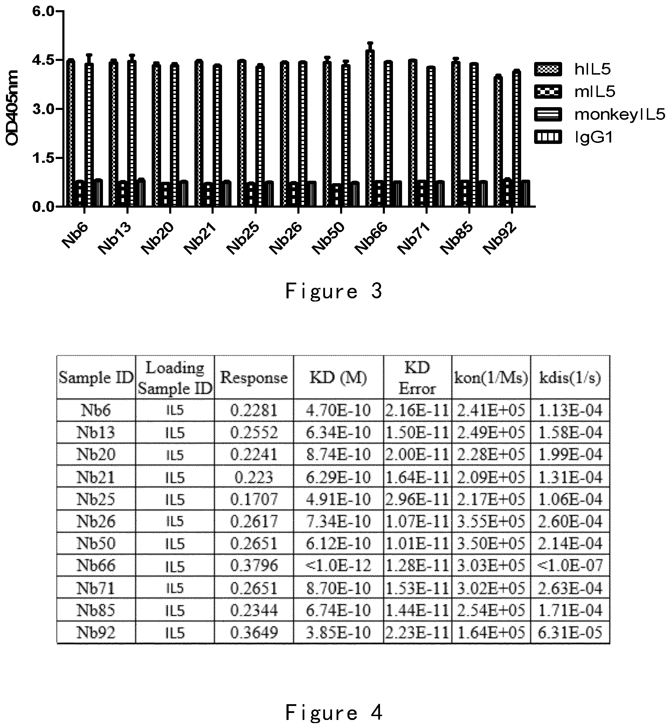

ELISA was used to detect whether the 11 nanobodies obtained in Example 2 could cross-react with other species of IL5. 1 µg/mL human IL5, mouse IL5, cynomolgus macaques IL5 and IgG1 proteins were respectively added to the enzyme label plate at 100uL/well, and coated overnight at 4°C. After washing 5 times with PBST, 300µL 1% BSA was added to each well for blocking at room temperature for 2 hours. After washing 5 times with PBST, 100µL 2 µg/mL prokaryotic expressed nanobodies (Nb6, Nb13, Nb20, Nb21, Nb25, Nb26, Nb50, Nb66, Nb71, Nb85, Nb92) were added respectively and incubated at 37°C for 1 hour. Then the plate was washed for 5 times with PBST, 100µL diluted mouse anti-HA antibody (1:2000 dilution) was added and incubated for 1 hour at 37°C. After washing 5 times with PBST, 100µL diluted alkaline phosphatase modified anti-mouse antibody (1:2000 dilution) was added and incubated at 37°C for 1 hour. Then the plate was washed with PBST for 5 times, developing solution was added, and the absorption value was measured at 405nm wavelength by a microplate reader. The results are shown in Figure 3: 11 nanobodies can recognize human and cynomolgus macaques IL5, but can not recognize mouse IL5.

### Example 4: Affinity determination of IL5 nanobody

The binding kinetics of 11 strains of nanobodies obtained in Example 2 against human IL5 were measured by a Bio-layer interferometry (BLI) using the Fortebio Red96 instrument. For kinetic measurement, IL5 antigen protein was diluted to 1.5 µg/mL with PBST buffer; 11 strains of IL5 nanobodies were diluted with PBST buffer with 2-fold gradient for six concentration gradients (30nM, 15nM, 7.5nM, 3.75nM, 1.88nM, 0.94nM), and the operating conditions of the instrument were set: temperature 30 °C, shake speed 1000rpm. Probes coated with Protein A were used to capture antibody, capture time 180s; binding to gradient diluted antigen, binding time 300s; dissociation time 360s; 10mM glycine (pH 1.7) was used to regenerate 3 times, each time for 5s. Fortebio Analysis version 9.0 was used for the analysis, the 1:1 binding model Global mode was fitted, and the binding rate (Kon), the dissociation rate (Kdis) and the dissociation constant KD were calculated. The results are shown in Figure 4, the affinity of all 11 nanobodies to IL5 is less than 1nM.

### Example 5: ELISA detection of binding activity of IL5 nanobody

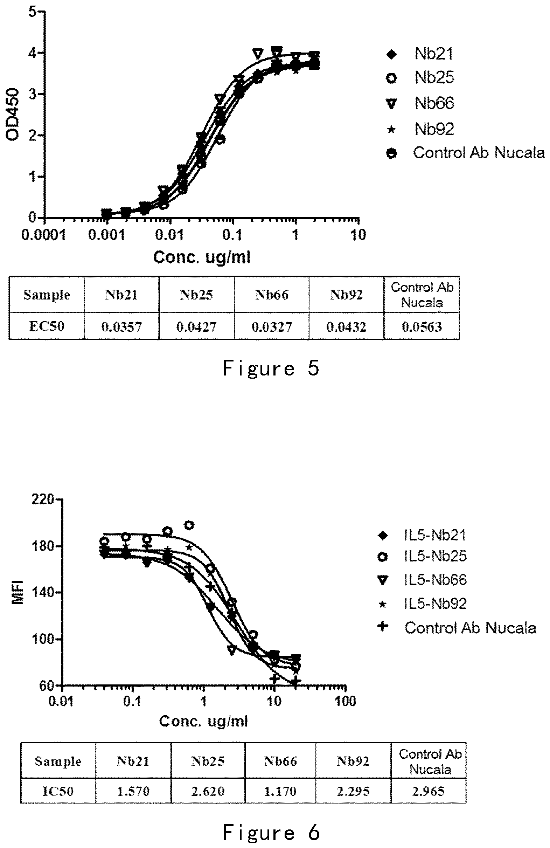

ELISA was used to detect and compare the binding activity of 4 nanobodies (Nb21, Nb25, Nb66 and Nb92) with IL5. All IL5 nanobodies were diluted to 1 µg/mL, and 100uL per well was taken for coating overnight at 4°C. After washing with PBST for 5 times, 300µL 1% BSA was added to each well and placed at 37°C for blocking for 2 hours. The plate was washed with PBST for 5 times, 100µL of gradient diluted ILS-biotin was added, with the concentration gradient starting at 2 µg/mL, and diluted at 2-fold gradient for 12 points, and the sample was added and incubated at 37°C for 1 hour. After washing with PBST for 5 times, 100µL of SA-HRP (diluted with PBS to 1:5000) was added and incubated at 37°C for 1 hour. The plate was washed with PBST for 5 times, 100µL TMB developing solution was added to each well, and reacted at room temperature in the dark for 5min, then 50µL 2M sulfuric acid was added to terminate the reaction, and the absorbance value was measured at 450nm by a microplate reader. The results are shown in Figure 5: the binding activity of the four nanobodies is superior to that of the control antibody Nucala.

### Example 6: Detection of blocking activity of IL5 antibody by flow cytometry

The HEK293F stable cells with high expression of IL5R were centrifuged at 1000rpm for 5min, and the supernatant was discarded. The cells were washed once with 5 mL PBS and then resuspended with 2mL PBS. After counting, the cells were divided into a 96-well plate with 3 × 10⁵ cells per well. Four nanobodies (Nb21, Nb25, Nb66 and Nb92) and control antibody Nucala were diluted in 2-fold gradient (20 µg/ml, 10 µg/ml, 5 µg/ml, 2.5 µg/ml, 1.25 µg/ml, 0.625 µg/ml, 0.31 µg/ml, 0.16 µg/ml, 0.08 µg/ml, 0.04 µg/ml, 0.02 µg/ml, 0.01 µg/ml), and the diluted antibodies were mixed with 2.5 µg/mL ILS-biotin in equal volume respectively to resuspend the cells in 96-well plate, and incubated at 4°C for 20 minutes. After centrifugation at 4°C at 3000rpm, 200µL PBS was added to each well, and then centrifuged at 3000rpm and 4°C for 4 min after resuspension. The diluted SA-PE antibody (diluted at 0.3:100) was added and incubated at 4°C for 20min. After centrifugation at 4°C for 4min at 3000rpm, the supernatant was discarded, and 200µL PBS was added to each well to wash the cells twice. Then 200µL PBS was added to resuspend the cells, transfer to a flow tube, and the PE signal of each sample was detected by flow cytometry. The results are shown in Figure 6, all the four nanobodies have good IL5/IL5R blocking activity, and the blocking activity of Nb21 and Nb66 is significantly better than that of the control antibody Nucala.

### Example 7: Antigen recognition epitope analysis of IL5 nanobody

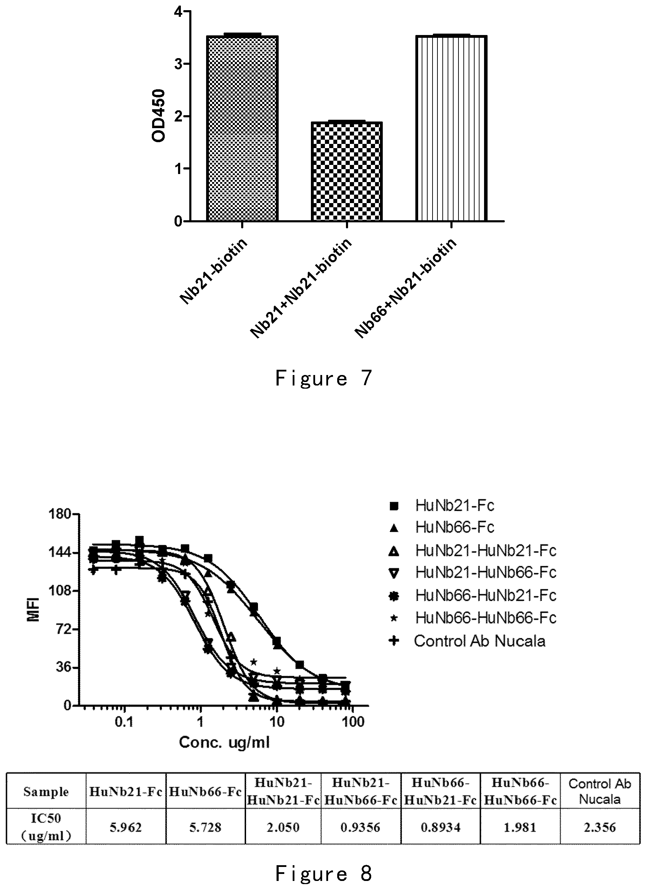

The ELISA method was used to detect whether the two nanobodies (Nb21 and Nb66) with better blocking activity in Example 6 recognized different IL5 epitopes. Specifically, 1 µg/mL IL5 antigen protein was coated overnight at 4°C. After washing the ELISA plate with PBST for 5 times, 1% BSA was added for blocking at room temperature for 2 hours. Then PBST was used to wash the ELISA plate for 5 times, and 100µL diluted antibody mixture was added and incubated at 37°C for 1 hour (the working concentration of nanobody is 20 µg/mL, the working concentration of the biotinylated nanobody is 3 µg/mL, and the three groups of samples are: 3 µg/mL Nb21-biotin, 20 µg/mL Nb21 +3 µg/mL Nb21-biotin, 20 µg/mL Nb66 +3 µg/mL Nb21-biotin). The ELISA plate was washed with PBST for 5 times, then 100µL SA-HRP (diluted at 1:5000) was added and incubated at 37°C for 1 hour. After washing with PBST for 5 times, 100µL TMB developing solution was added to each well, and reacted at room temperature in the dark for 5min, then 50µL 2M sulfuric acid was added to terminate the reaction, and the absorbance value was measured at 450nm by a microplate reader. The results are shown in Figure 7: Nb21 and Nb66 have different antigen recognition epitopes.

### Example 8: Construction and expression of bivalent humanized nanobodies

The two strains of nanobodies Nb21 and Nb66 with good blocking activity in the above-mentioned Example 6 were respectively subjected to the humanization transformation of the skeleton region, and the transformation scheme can be found in the Example 3 of the patent CN110144010B. The humanized antibody sequences are shown in Table 2.

**Table 2 Sequence listing of humanized IL5 nanobodies**

| Antibod y No. | Amino acid sequence of variable region | | | Amino acid sequence of framework region | | | | Full-lengt h amino acid sequence | Full-lengt h base sequence |
|---|---|---|---|---|---|---|---|---|---|
| | CDR1 | CDR2 | CDR3 | FR1 | FR2 | FR3 | FR4 | | |
| HuNb21 | SEQ ID NO:1 | SEQ ID NO:2 | SEQ ID NO:3 | SEQ ID NO:37 | SEQ ID NO:38 | SEQ ID NO:39 | SEQ ID NO:40 | SEQ ID NO:41 | SEQ ID NO:42 |
| HuNb66 | SEQ ID NO:19 | SEQ ID NO:20 | SEQ ID NO:21 | SEQ ID NO:43 | SEQ ID NO:44 | SEQ ID NO:45 | SEQ ID NO:46 | SEQ ID NO:47 | SEQ ID NO:48 |

The humanized antibodies were combined in pairs and expressed in fusion with Fc, and were constructed in pCDNA3.1+ vector. The sequences after fusion are shown in Table 3.

**Table 3 Sequence listing of humanized IL5 nanobodies**

| Antibody structure | Amino acid sequence | Base sequence |
|---|---|---|
| HuNb21-Fc | SEQ ID NO:49 | SEQ ID NO:50 |
| HuNb66-Fc | SEQ ID NO:51 | SEQ ID NO:52 |
| HuNb21-HuNb21-Fc | SEQ ID NO:53 | SEQ ID NO:54 |
| HuNb21-HuNb66-Fc | SEQ ID NO:55 | SEQ ID NO:56 |
| HuNb66-HuNb66-Fc | SEQ ID NO:57 | SEQ ID NO:58 |
| HuNb66-HuNb21-Fc | SEQ ID NO:59 | SEQ ID NO:60 |

The constructed plasmids were transfected into HEK293F cells. The expression method is shown in Example 3 of patent CN2018101517526.

### Example 9: Detection of Blocking Activity of Bivalent Humanized IL5 Nanobody by Flow Cytometry

The purified humanized bivalent antibodies obtained in Example 8 were compared to the monovalent nanobodies and the positive control Nucala for blocking activity at the cellular level. Specifically, The HEK293F stable cells with high expression of IL5R were centrifuged at 1000rpm for 5 min, and the supernatant was discarded. The cells were washed once with 5 mL PBS and then resuspended with 2mL PBS. After counting, the cells were divided into a 96-well plate with 3 × 10⁵ cells per well. The antibodies to be tested were diluted in 2-fold gradient (80µg/ml, 40µg/ml, 20 µg/ml, 10 µg/ml, 5 µg/ml, 2.5 µg/ml, 1.25 µg/ml, 0.625 µg/ml, 0.31 µg/ml, 0.16 µg/ml, 0.08 µg/ml, 0.04 µg/ml), and the diluted antibodies were mixed with IL5-biotin respectively to resuspend the cells in 96-well plate, and incubated at 4°C for 20 minutes. After centrifugation at 4°C at 3000rpm, 200µL PBS was added to each well, and then centrifuged at 3000rpm and 4°C for 4 min after resuspension. The diluted SA-PE antibody (diluted at 0.3:100) was added and incubated at 4°C for 20min. After centrifugation at 4°C for 4 min at 3000rpm, the supernatant was discarded, and 200µL PBS was added to each well to wash the cells twice. Then 200µL PBS was added to resuspend the cells, transfer to a flow tube, and the PE signal of each sample was detected by flow cytometry.

The results are shown in Figure 8: the activity of the humanized bivalent antibody is significantly higher than that of the monovalent antibody, and the blocking activity of the HuNb21-HuNb66-Fc and HuNb66-HuNb21-Fc heterologous bivalent antibodies is better, and is significantly superior to that of the control antibody Nucala.

### Example 10: Expression of Bivalent Biepitope IL5 Nanobody in Pichia pastoris

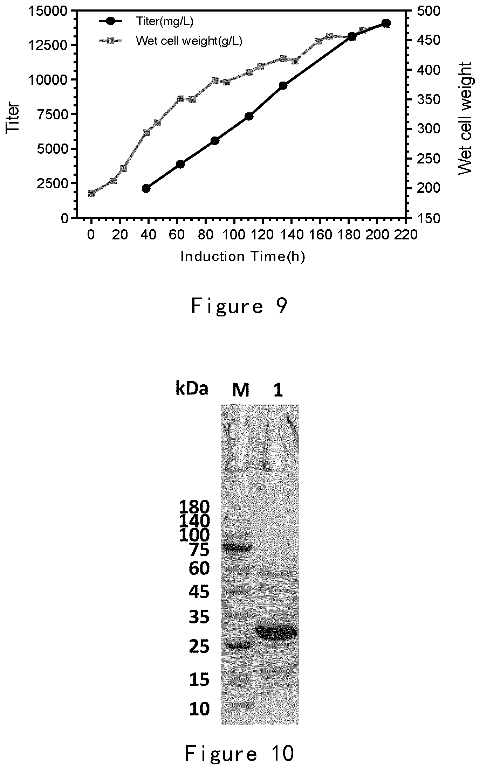

Preferably, the above humanized Nb66 and Nb21 are combined to form a bivalent biepitope nanobody, and the amino acid sequence of the antibody is shown in SEQ ID NO: 61. After optimizing the codon in Pichia pastoris, the base sequence is shown in SEQ ID NO:62. The sequence was cloned into a pPICZaA vector, and then expressed by Pichia pastoris. Briefly, the expression method is as follows: the pPICZaA-HuNb66-HuNb21 was linearized with Sac I restriction endonuclease and electrotransformed into X-33 competent cells. The electroporated samples were respectively coated on YPD plate medium containing different concentrations of bleomycin resistance, and cultured in an incubator at 30°C for 3-4 days. The specific implementation scheme can be found in the instructions of pPICZaA vector provided by Invitrogen Company. After a monoclone grows on the plate medium, the monoclone on plates with different concentrations was selected and placed in BMGY culture medium. When the OD value of BMGY culture medium reached about 20, the bacteria were collected and replaced in BMMY culture medium, and cultured at 28°C, 250rpm. Thereafter, the sample was taken every 24 hours, and methanol with a final volume of 1% was added and sampled. The sample was centrifuged at 12000rpm for 5min, and the supernatant was taken and stored at -20°C. After continuous induction for 5 days, the culture was terminated, and the supernatant was taken to determine the target protein content. Subsequently, a high-yield clone was selected for 7L fermenter culture. The fermentation conditions were 24°C induction culture, pH 6.5, and methanol feeding rate was 8.5mL/L/h. The supernatant was taken at different time points in the fermentation process to detect the target protein content, and the results are shown in Figure 9: the expression yield of the dual-epitope IL5 nanoantibody HuNb66-HuNb21 in Pichia pastoris can reach about 13g/L. The obtained sample was diluted 13 times for SDS-PAGE detection, and the results are shown in Figure 10: the target protein is clear, and the purity of the expression supernatant is high.

**Example 11: Detection of blocking activity of bivalent biepitope IL5 nanobody**

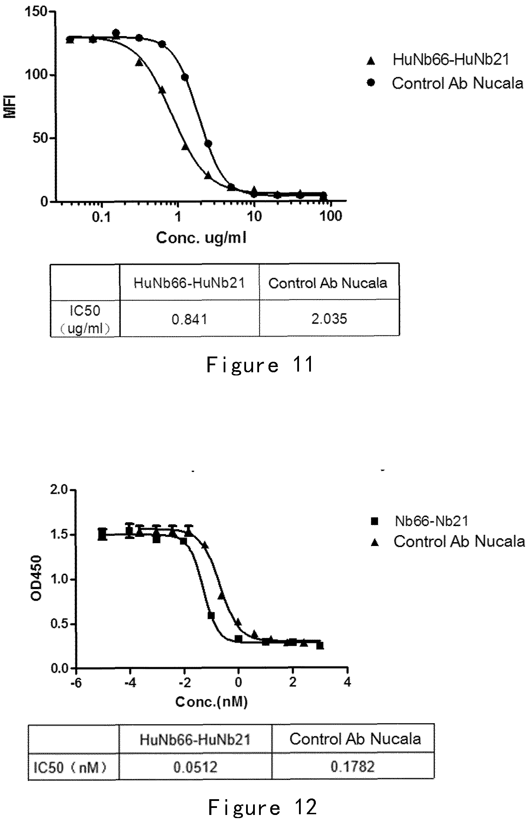

The above bivalent biepitope antibody expressed by yeast was purified by Protein A affinity chromatography to obtain a higher purity antibody, and then the blocking activity was detected. The detection method was the same as in Example 9. The results are shown in Figure 11, the blocking activity of the bivalent biepitopic antibody HuNb66-HuNb21 expressed by yeast (IC₅₀= 0.841 µg/mL) is better than that of the control antibody Nucala (IC₅₀= 2.035 µg/mL).

### Example 12: The proliferation inhibitory effect of the bivalent biepitope IL5 nanobody on TF1 cells

The resuscitated TF-1 cells ( treated by IL5 induction) were centrifuged at 1000 rpm for 5 min, and the supernatant was discarded. The cells were resuspended with 5mL PBS and centrifuged at 1000 rpm for 5 min. 20mL 1640 medium was used to resuspend the cells for counting, and the concentration of the cell solution was diluted to 6 × 10⁵/mL, and the cells were divided into a 96-well plate at 60uL/well. After mixing SOuL gradient diluted IL5 antibody with SOuL IL5 protein of 25ng/mL, respectively, 40uL mixed solution was added to the cell solution. At the same time, 200uL PBS was added to all cell peripheral wells to prevent evaporation of solution in cell wells, and the cells were cultured in a 5% CO₂ incubator at 37°C for 72 hours. The cell culture plate was taken out, and CCK8 solution was added at 10uL/well, and placed at 37°C for developing for 4 hours. After completion of development, the OD450 value of each well was read on a microplate reader.

The results are shown in Figure 12: the bivalent biepitope antibody expressed by yeast can effectively inhibit the proliferation of TF-1 cells induced by IL5, and its inhibitory activity (IC_{50 HuNb66-HuNb21} = 0.0512 nM) is better than that of the control antibody on the proliferation of TF-1 cells (IC_{50 Nucala} = 0.1782 nM).

All references mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

## Claims

1. An anti-IL5 nanobody, which can specifically bind to IL5, and the complementary determination region (CDR) of the VHH chain in the nanobody is one or more selected from the group consisting of:
(1) CDR1 shown in SEQ ID NO: 19, CDR2 shown in SEQ ID NO:20, and CDR3 shown in SEQ ID NO:21;
(2) CDR1 shown in SEQ ID NO:1, CDR2 shown in SEQ ID NO:2, and CDR3 shown in SEQ ID NO:3;
(3) CDR1 shown in SEQ ID NO: 10, CDR2 shown in SEQ ID NO: 11, and CDR3 shown in SEQ ID NO: 12; and
(4) CDR1 shown in SEQ ID NO:28, CDR2 shown in SEQ ID NO:29, and CDR3 shown in SEQ ID NO:30.

2. The anti-IL5 nanobody of claim 1, wherein the VHH chain of the anti-IL5 nanobody further comprises a framework region (FR), and the framework region (FR) is one or more selected from the group consisting of:
(1) FR1 shown in SEQ ID NO:4, FR2 shown in SEQ ID NO:5, FR3 shown in SEQ ID NO:6, and FR4 shown in SEQ ID NO:7;
(2) FR1 shown in SEQ ID NO: 13, FR2 shown in SEQ ID NO: 14, FR3 shown in SEQ ID NO:15, and FR4 shown in SEQ ID NO: 16;
(3) FR1 shown in SEQ ID NO:22, FR2 shown in SEQ ID NO:23, FR3 shown in SEQ ID NO:24, and FR4 shown in SEQ ID NO:25;
(4) FR1 shown in SEQ ID NO:31, FR2 shown in SEQ ID NO:32, FR3 shown in SEQ ID NO:33, and FR4 shown in SEQ ID NO:34;
(5) FR1 shown in SEQ ID NO:37, FR2 shown in SEQ ID NO:38, FR3 shown in SEQ ID NO:39, and FR4 shown in SEQ ID NO:40; and
(6) FR1 shown in SEQ ID NO:43, FR2 shown in SEQ ID NO:44, FR3 shown in SEQ ID NO:45, and FR4 shown in SEQ ID NO:46.

3. The anti-IL5 nanobody of claim 1, wherein the amino acid sequence of the VHH chain of the anti-IL5 nanobody is selected from the group consisting of: SEQ ID NO: 26, SEQ ID NO: 47, SEQ ID NO: 8, SEQ ID NO: 41, SEQ ID NO: 17, SEQ ID NO: 35, and a combination thereof.

4. An anti-IL5 antibody, wherein the antibody comprises one or more anti-IL5 nanobodies of claim 1.

5. The antibody of claim 4, wherein the antibody comprises a monomer, a bivalent antibody, and/or a multivalent antibody.

6. The antibody of claim 4, wherein the anti-IL5 antibody comprises one or more VHH chains with amino acid sequences as shown in SEQ ID NO: 26, SEQ ID NO: 47, SEQ ID NO: 8, SEQ ID NO: 41, SEQ ID NO:41 or SEQ ID NO:35.

7. An anti-IL5 nanobody Fc fusion protein, wherein the structure of the fusion protein from N-terminus to C-terminus is as shown in the Formula Ia or Ib:
A- L-B (Ia)
;
B- L-A (Ib)
; wherein,
A is one or more anti-IL5 nanobodies of claim 1;
B is the Fc fragment of IgG; and
L is none or a flexible linker.

8. The fusion protein of claim 7, wherein the amino acid sequence of the fusion protein is as shown in SEQ ID NO:49, SEQ ID NO:51, SEQ ID NO:53, SEQ ID NO:55, SEQ ID NO:57, or SEQ ID NO:59.

9. A polynucleotide, which encodes a protein selected from the group consisting of the anti-IL5 nanobody of claim 1, the anti-IL5 antibody of claim 4, or the anti-IL5 nanobody Fc fusion protein of claim 7.

10. An expression vector comprising the polynucleotide of claim 9.

11. A host cell comprising the expression vector of claim 10, or having the polynucleotide of claim 7 integrated into the genome thereof.

12. A method for producing an anti-IL5 nanobody, an anti-IL5 antibody or the Fc fusion protein thereof, which comprises the steps:
(a) culturing the host cell of claim 11 under conditions suitable for producing the nanobody or Fc fusion protein thereof, thereby obtaining a culture containing the anti-IL5 nanobody or Fc fusion protein thereof;
(b) isolating or recovering the anti-IL5 nanobody or Fc fusion protein thereof from the culture; and
(c) optionally, purifying and/or modifying the anti-IL5 nanobody or Fc fusion protein thereof obtained in step (b).

13. An immunoconjugate containing:
(a) the anti-IL5 nanobody of claim 1, or the anti-IL5 antibody of claim 4, or the anti-IL5 nanobody Fc fusion protein of claim 7; and
(b) a coupling moiety selected from the group consisting of a detectable label, a drug, a toxin, a cytokine, a radionuclide, an enzyme, a gold nanoparticle/nanorod, a nanomagnetic particle, a viral coat protein or VLP, and a combination thereof.

14. A pharmaceutical composition containing:
(i) the anti-IL5 nanobody of claim 1, or the anti-IL5 antibody of claim 4, or the anti-IL5 nanobody Fc fusion protein of claim 7, or the immunoconjugate of claim 13; and
(ii) a pharmaceutically acceptable carrier.

15. Use of the anti-IL5 nanobody of claim 1, the anti-IL5 antibody of claim 4, the anti-IL5 nanobody Fc fusion protein of claim 7, or the immunoconjugate of claim 13; (a) for the preparation of a medicament for the prevention and/or treatment of a diseases or conditions related to IL5/IL5R signaling; (b) for the preparation of a reagent, a test plate or a kit for detecting IL5.
